# EUROPÄISCHE PATENTSCHRIFT

(11) **EP 4 442 101 B1**
(45) Veröffentlichungstag und Bekanntmachung des Hinweises auf die Patenterteilung: **06.05.2026**
(21) Anmeldenummer: 24157250.2
(22) Anmeldetag: 13.02.2024
(51) Int. Cl.: A01D 41/127, G01N 27/22

(54) **SENSORANORDNUNG**
SENSOR ARRANGEMENT
AGENCEMENT DE CAPTEUR

(30) Priorität: 05.04.2023 DE 102023108762
(43) Veröffentlichungstag der Anmeldung: 09.10.2024
(73) Patentinhaber: CLAAS Selbstfahrende Erntemaschinen GmbH, 33428 Harsewinkel (DE)
(72) Erfinder: Neitemeier, Dennis, 59510 Lippetal (DE); Lehmann, Christoph, 49191 Belm (DE)
(74) Vertreter: CLAAS Gruppe

(56) Entgegenhaltungen:
- EP-A1- 0 908 087
- EP-B1- 3 479 673
- DE-C2- 19 610 599
- US-B2- 10 729 067
- US-B2- 11 016 049

## Beschreibung

Die vorliegende Erfindung betrifft eine Sensoranordnung gemäß dem Oberbegriff des Anspruches 1. Weiterhin ist sie eine selbstfahrende Erntemaschine gemäß dem Oberbegriff des Anspruches 5 Gegenstand der vorliegenden Erfindung. Aus der EP 3 479 673 B1 ist eine Sensoranordnung für eine selbstfahrende Erntemaschine gemäß dem Oberbegriff des Anspruches 1 bekannt. Zum Schutz der auf dem plattenförmigen Träger angeordneten Sensorelemente vor der abrasiven Wirkung des die Sensorelemente überströmenden Erntegutes ist die Kontaktfläche mit einer dielektrischen Schutzbeschichtung versehen. Die Schutzbeschichtung umfasst einen keramischen Füllstoff, der in eine Polymer- oder Kunststoffmatrix oder ein Harz gebunden ist. Neben einer Erhöhung des Abstands zwischen den Sensorelementen und dem Erntegut durch die Aufbringung der Schutzbeschichtung kann der Prozess der Aufbringung Toleranzen unterliegen, wodurch es zu einem erhöhten Kalibrieraufwand kommt. Ein weiterer Aspekt besteht in der Feuchtigkeitsabsorption der Polymer- oder Kunststoffmatrix bzw. des Harzes, was zu Messunsicherheiten führen kann.

Weitere Sensoranordnungen sind aus der DE 196 10 599 C2 und der EP 0 908 087 A1 bekannt.

Ausgehend vom vorstehend genannten Stand der Technik ist es demnach die Aufgabe der vorliegenden Erfindung, eine Sensoranordnung der eingangs genannten Art weiterzubilden, welche die Nachteile des Standes der Technik vermeidet sowie dass die Sensoranordnung durch einen bauraumeffizienteren Aufbau in einer selbstfahrenden Erntemaschine flexibler einsetzbar ist.

Diese Aufgabe wird erfindungsgemäß durch die Merkmale des unabhängigen Patentanspruchs 1 gelöst, wobei vorteilhafte Weiterbildungen der erfindungsgemäßen Sensoranordnung Gegenstand der entsprechenden abhängigen Patentansprüche 2 bis 4 sind.

Gemäß Anspruch 1 wird eine Sensoranordnung zur Erfassung wenigstens einer Guteigenschaft eines von einer selbstfahrenden Erntemaschine aufgenommenen und diese durchlaufenden Erntegutes vorgeschlagen, umfassend zumindest ein Sensorelement, welches auf einem plattenförmigen Träger angeordnet ist, sowie eine Auswerteschaltung zur Auswertung von Signalen, welche das zumindest eine Sensorelement aufgrund eines physischen Kontakts des Trägers mit dem Erntegut generiert. Erfindungsgemäß ist vorgesehen, dass der Träger als eine keramikhaltige Platte mit einer Kontaktfläche, welche im Kontakt mit dem Erntegut steht, und einer der Kontaktfläche abgewandten Sensorfläche ausgeführt ist, wobei auf der dem Erntegut abgewandten Sensorfläche des Trägers das zumindest eine Sensorelement und die Auswerteschaltung an dieser angeordnet sind. Die Sensoranordnung besteht lediglich aus einem einzelnen Träger, welcher direkt in einen Gutfluss montiert werden kann, ohne dass es des Einsatzes einer Schutzbeschichtung oder einer sonstigen Schutzmaßnahme bedarf. Zudem zeichnet sich die erfindungsgemäße Sensoranordnung durch eine geringere Bauhöhe aus, wodurch die Anordnung flexibler wird und weitere Einsatzpositionen und Einsatzmöglichkeiten eröffnet werden. Ein weiterer Vorteil bei der Ausführung des Trägers als keramikhaltige Platte, wobei sie vorzugsweise als Keramik ausgestaltet ist, besteht in den herausragenden elektrischen Eigenschaften bis in hohe Frequenzbereiche, was eine Auswertung der Permittivität auch in diesen Frequenzbereichen ermöglicht.

Insbesondere kann das zumindest eine Sensorelement als zwei zueinander beabstandete Elektroden ausgeführt sein. Durch den reduzierten Abstand zwischen Erntegut und den als Elektroden ausgeführten Sensorelementen nimmt die Sensitivität der Sensoranordnung zu und die Auflösung bei einer lokalen Erfassung der Permittivität kann erhöht werden. Die notwendigen Elektroden können verkleinert oder die Anforderungen an die Auswerteelektronik kann gesenkt werden.

Bevorzugt können das zumindest eine Sensorelement und die Auswerteschaltung als eine Leiterplatine ausgeführt sein. Eine direkte Bestückung des zumindest einen Sensorelements und der Auswerteschaltung auf dem Träger führt zum Wegfall von Bondingschritten. Mit einem Hintergießen des Trägers auf der der Kontaktfläche abgewandten Sensorfläche ist eine direkte Anordnung in der Erntemaschine denkbar.

Insbesondere kann beabstandet zum plattenförmigen Träger ein elektrischer Anschluss in einem den Träger aufnehmenden Sensorgehäuse angeordnet sein, um eine Leitung an die Sensoranordnung anzuschließen. Der Träger kann dabei durch Einkleben in das Sensorgehäuse befestigt sein.

Weiterhin wird die eingangs gestellte Aufgabe durch eine selbstfahrende Erntemaschine gemäß Anspruch 5 gelöst.

Gemäß Anspruch 5 wird eine selbstfahrende Erntemaschine vorgeschlagen, welche zur Aufnahme und Bearbeitung von Erntegut mittels verschiedener an oder in der Erntemaschine angeordneter Arbeitsorgane eingerichtet ist, wobei in der Erntemaschine zumindest eine mit dem Erntegut in physischen Kontakt kommende Sensoranordnung zur Erfassung zumindest eines Erntegutparameters angeordnet ist, wobei die zumindest eine Sensoranordnung nach einem der vorangehenden Ansprüche 1 bis 5 ausgebildet ist. Auf die Vorteile der erfindungsgemäßen Sensoranordnung darf verwiesen werden. Bevorzugt ist die zumindest eine Sensoranordnung als kapazitive Sensoranordnung ausgeführt.

Insbesondere kann die Erntemaschine als Feldhäcksler oder als Mähdrescher ausgeführt sein. Während die als Feldhäcksler ausgeführte Erntemaschine im Wesentlichen feuchtes Erntegut verarbeitet, verarbeitet die als Mähdrescher ausgeführte Erntemaschine im Wesentlichen trockenes Erntegut. In beiden Fällen ist die Anordnung der wenigstens einen erfindungsgemäßen Sensoranordnung zur Erfassung zumindest eines Erntegutparameters wegen der verbesserten Abrasionsbeständigkeit und des geringen Bauraumbedarfs, insbesondere der geringen Bauhöhe, vorteilhaft.

Gemäß einer Weiterbildung kann die zumindest eine Sensoranordnung in wenigstens einer Wandung eines Einzugskanals, eines Förderschachts und/oder eines Auswurfkrümmers des Feldhäckslers angeordnet sein.

Gemäß einer Weiterbildung kann die zumindest eine Sensoranordnung in wenigstens einer Wandung eines Schrägförderers, eines Kornelevators und/oder eines Überkehrelevators des Mähdreschers angeordnet sein und/oder kann die zumindest eine Sensoranordnung einer Dreschvorrichtung, einer Abscheidevorrichtung und/oder einer Reinigungsvorrichtung des Mähdreschers zugeordnet sein. Insbesondere kann die zumindest eine Sensoranordnung hinter einem siebartigen Element der Dreschvorrichtung, der Abscheidevorrichtung und/oder der Reinigungsvorrichtung angeordnet sein. Dabei kann das siebartige Element ein Dreschkorb, ein Sieb der Abscheidevorrichtung, ein Reinigungssieb sein oder als hinter einem Reinigungssieb angeordnete rechenförmig angeordnete Finger ausgeführt sein.

Insbesondere kann die zumindest eine Sensoranordnung zur Detektion einer Erntegutfeuchtigkeit, eines Erntegutdurchsatzes, einer Erntegutdichte und/oder einer Querverteilung eingerichtet sein. Zumindest zur Detektion der Querverteilung können mehrere Sensoranordnungen beispielsweise über die Breite der Abscheidevorrichtung oder eines Reinigungssiebes verteilt hinter oder unter diesen angeordnet sein.

Bevorzugt kann der Träger der Sensoranordnung in ein Sensorgehäuse oder unmittelbar in eine mit dem Erntegut in Berührung kommende Fläche eines der Arbeitsorgane integriert sein. Eine mit dem Erntegut in Berührung kommende Fläche eines der Arbeitsorgane kann eine Wandung des Schrägförderers, des Kornelevators, des Überkehrelevators, des Einzugskanals, des Förderschachts und/oder des Auswurfkrümmers sein.

Des Weiteren wird die eingangs gestellte Aufgabe durch ein Verfahren zum Herstellen einer erfindungsgemäßen Sensoranordnung mit den Merkmalen des Anspruches 13 gelöst.

Des Weiteren wird ein Verfahren zur Herstellung einer Sensoranordnung vorgeschlagen, umfassend die Verfahrensschritte:
- Herstellen eines aus einem keramischen Material bestehenden plattenförmigen Trägers mit einer Kontaktfläche und einer der Kontaktfläche gegenüberliegenden Sensorfläche,
- Aufbringen einer Kupferschicht auf der Oberfläche der Sensorfläche des plattenförmigen Trägers durch einen chemischen Bearbeitungsprozess, und
- Bearbeiten der Kupferschicht zur Ausbildung zumindest eines Sensorelements und einer Auswerteschaltung.

Die gemäß dem Verfahren hergestellte Sensoranordnung hat den Vorteil, dass diese eine Anordnung in Bereichen einer selbstfahrenden Erntemaschine ermöglicht, die bislang aufgrund ihrer Einbauhöhe von aus dem Stand der Technik bekannten Sensoranordnungen nicht zugänglich sind.

Insbesondere kann das Bearbeiten der Kupferschicht, in welcher das zumindest eine Sensorelement und die Auswerteschaltung ausgebildet werden, bevorzugt durch Ätzen oder Laserablation erfolgen.

Die vorliegende Erfindung wird nachstehend anhand eines in den Zeichnungen dargestellten Ausführungsbeispieles näher erläutert.

Es zeigen:
- Fig. 1: eine schematische Darstellung einer als selbstfahrender Feldhäcksler ausgeführten landwirtschaftlichen Erntemaschine in Seitenansicht;
- Fig. 2: eine schematische Darstellung einer als selbstfahrender Mähdrescher ausgeführten landwirtschaftlichen Erntemaschine in Seitenansicht;
- Fig. 3: schematisch und exemplarisch einen Prinzipaufbau einer aus dem Stand der Technik bekannten Sensoranordnung; und
- Fig. 4: schematisch und exemplarisch einen Prinzipaufbau einer erfindungsgemäßen Sensoranordnung.

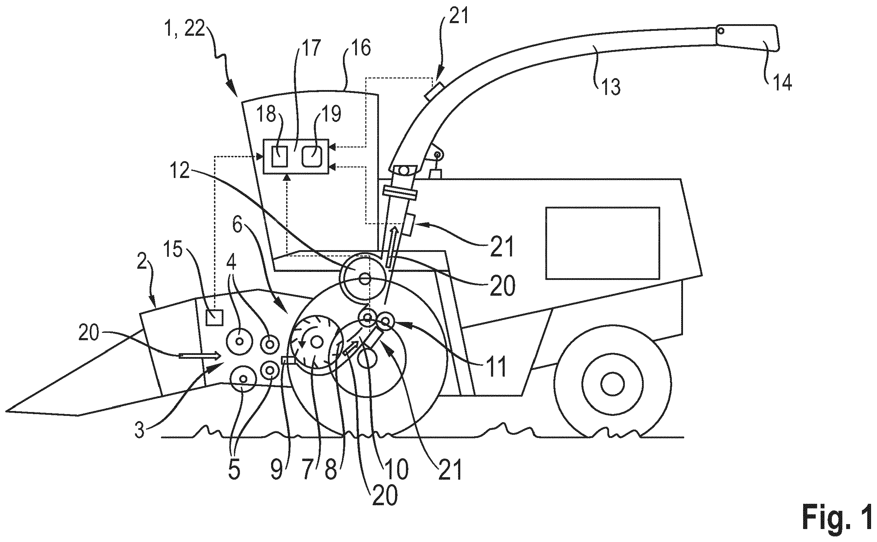

In Fig. 1 ist eine als Feldhäcksler 22 ausgeführte selbstfahrende landwirtschaftliche Erntemaschine 1 abgebildet, an welcher im frontseitigen Bereich zur Aufnahme von auf dem Boden abgelegtem Erntegut ein Vorsatzgerät 2 angeordnet ist. Das Vorsatzgerät 2 variiert in Abhängigkeit von der zu erntenden bzw. aufzunehmenden Erntegutart. Das Vorsatzgerät 2 nimmt das Erntegut vom Feld auf und fördert es einem Einzugsorgan 3 zu, das im dargestellten Ausführungsbeispiel aus einer Walzengruppe mit oberen und unteren Einzugswalzen 4, 5 besteht. Die Einzugswalzen 4, 5 des Einzugsorgans 3 üben eine Presskraft auf das aufgenommene Erntegut aus. Das Einzugsorgan 3 fördert das zu einer Erntegutmatte verdichtete Erntegut einer Häckselvorrichtung 6 zu, welche eine rotierend angetriebene Häckseltrommel 7 mit über deren Umfang verteilt angeordneten Häckselmessern 8 aufweist. Die Häckselmesser 8 schneiden die von dem Einzugsorgan 3 zugeführte Erntegutmatte an einer Gegenschneide 9. Ein geschnittener bzw. gehäckselter Erntegutstrom 20 gelangt durch die Rotationsbewegung der Häckseltrommel 7 in einen nachgeordneten Förderkanal 10, von wo es je nach Ausstattung des Feldhäckslers 1 durch eine optional im Gutflussweg angeordnete Nachbearbeitungsvorrichtung 11, auch als Konditioniervorrichtung oder Corncracker bezeichnet, aufbereitet und von einem nachgeordneten, rotierend angetriebenen Nachbeschleunigungsorgan 12 zusätzlich beschleunigt durch eine verstellbare Überladeeinrichtung 13 in ein mitgeführtes Transportfahrzeug gefördert wird. Die optionale Nachbearbeitungsvorrichtung 11 kann wahlweise aus dem Gutflussweg ausgeschwenkt oder vollständig entfernt werden. Die Überladeeinrichtung 13 ist um eine vertikal verlaufende Achse drehbar, beispielsweise mittels eines Drehkranzes. Zusätzlich und unabhängig davon ist die Überladeeinrichtung 13 um eine horizontal verlaufende Achse schwenkbar. Am freien Ende der Überladeeinrichtung 13 kann eine sogenannte Auswurfklappe 14 angeordnet sein, welche gegenüber der Überladeeinrichtung 13 um eine horizontal verlaufende Achse schwenkbar ist.

Zum Antreiben der Arbeitsorgane der Erntemaschine 1, d.h. von Vorsatzgerät 2, Einzugsorgan 3, Häckselvorrichtung 6, optional der Nachbearbeitungsvorrichtung 11, sowie dem Nachbeschleunigungsorgan 12, ist ein Antriebsmotor 19 vorgesehen. Die Arbeitsorgane können durch einen - nicht dargestellten - Hauptantriebsstrang, mit der Motorabtriebswelle des Antriebsmotors verbunden sein. Der Antriebsmotor dient auch zum Betreiben eines hydrodynamischen Fahrantriebs des Feldhäckslers 1.

Das Einzugsorgan 3 kann einen Schichthöhensensor 15 aufweisen. Mittels des Schichthöhensensors 15 lässt sich das Vorhandensein von Erntegut und der Durchsatz an aufgenommenem Erntegut bestimmen.

Die Erntemaschine 1 umfasst eine Fahrerkabine 16, in der eine Eingabe-Ausgabe-Einheit angeordnet ist. Weiterhin umfasst die Erntemaschine 1 eine Steuerungsvorrichtung 17, welche eine Speichereinheit 18 zum Hinterlegen von Daten und eine Recheneinheit 19 zur Verarbeitung der in dem Speichereinheit 18 hinterlegten Daten umfasst. Die Steuerungsvorrichtung 17 ist dazu eingerichtet, einen Bediener der Erntemaschine 1 bei der Bedienung derselben zu unterstützen. Die Steuerungsvorrichtung 17 ist dabei zur Ansteuerung der verschiedenen Arbeitsorgane der Erntemaschine 1 eingerichtet.

Im Förderkanal 10 entlang des Gutflussweges 20 können eine oder mehrere Sensoranordnungen 21 vorgesehen sein. Die zumindest eine Sensoranordnung 21 ist als kapazitive Sensoranordnung ausgeführt. Die zumindest eine Sensoranordnung 21 kann zur Detektion einer Erntegutfeuchtigkeit, eines Erntegutdurchsatzes, einer Erntegutdichte und/oder einer Querverteilung eingerichtet sein.

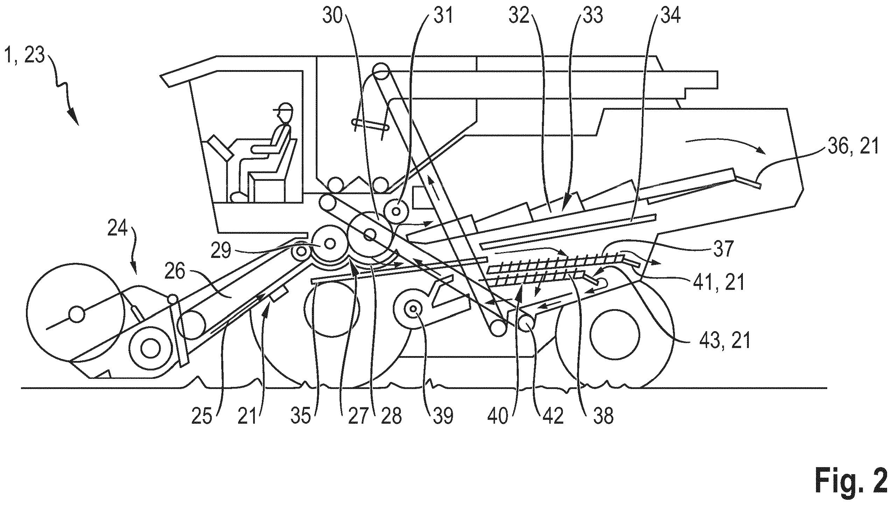

Fig. 2 zeigt schematisch eine als Mähdrescher 23 ausgeführte selbstfahrende landwirtschaftliche Erntemaschine 1. Der Mähdrescher 1 weist eine Vielzahl von Arbeitsorganen zur Förderung und/oder Verarbeitung von abzuerntendem Erntegut (nicht dargestellt) auf.

Das Erntegut wird mittels eines Vorsatzgerätes 24 aufgenommen und in einem zugeführten Erntegutstrom 25 (als Pfeil dargestellt) mittels eines Schrägförderers 26 einer Dreschvorrichtung 27 zugeführt. Die Dreschvorrichtung 27 umfasst einen Dreschkorb 28, eine Beschleunigungstrommel 29, eine Dreschtrommel 30 und eine Umlenktrommel 31. Am Dreschkorb 28 erfolgt eine erste Abscheidung von freibeweglichen Körnern aus dem Erntegutstrom 25 auf einen Vorbereitungsboden 35.

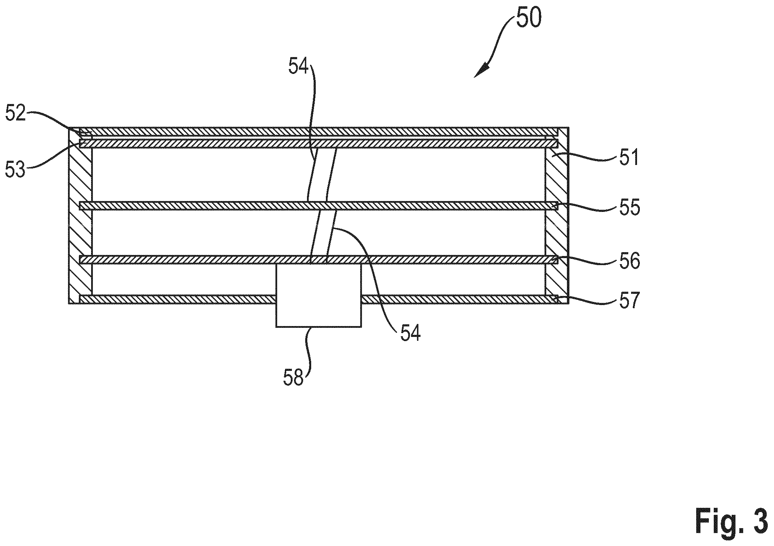

Nach Durchlaufen der Dreschvorrichtung 27 wird ein aus diesem austretender Erntegutstrom, der Halmteile und noch nicht ausgedroschene Körner enthält, einer als Hordenschüttler 32 ausgeführten Abscheidevorrichtung 33 zugeführt. Die im Erntegutstrom noch enthaltenen freibeweglichen Körner werden mittels des Hordenschüttlers 32 in Form eines weiteren Erntegutstroms auf einen Rücklaufboden 34 abgeschieden. Ein von der Abscheidevorrichtung 33 kommender Erntegutreststrom, vornehmlich bestehend aus Halmteilen, wird aus der Erntemaschine 23 herausgefördert. Der Erntegutreststrom überquert dabei einen Verlustkornzähler 36. Der Verlustkornzähler 36 ist als Sensoranordnung 21, wie sie in Figur 3 dargestellt ist, ausgeführt. Ein Kondensator, bevorzugt ein planarer Interdigitalkondensator, ist auf einer flachen Oberfläche im Auslaufbereich der Abscheidevorrichtung 33 angeordnet, über welche der vierte Erntegutstrom hinweg fließt. Die Sensoranordnung 21 erfasst die Elemente des Erntegutstromes und unterscheidet dabei zwischen Korn- und Nichtkornelementen.

Der Mähdrescher 23 kann anstelle des Hordenschüttlers 32 eine als Axial-Trennrotoren ausgeführte Abscheidevorrichtung 33 aufweisen.

Sowohl der aus dem Dreschkorb 28 als auch der aus der Abscheidevorrichtung 33 austretende, vornehmlich Körner enthaltende Erntegutstrom werden über den Rücklaufboden 34 und den Vorbereitungsboden 45 zu einem Erntegutstrom zusammengeführt und einer aus mehreren Siebebenen 37, 38 und einem Gebläse 39 bestehenden Reinigungsvorrichtung 40 zugeführt. Die Körner dieses Erntegutstroms werden hier gereinigt und Nicht-Kornbestandteile, wie beispielsweise Spreu- und Halmteile, in Form eines weiteren Erntegutreststroms abgetrennt und aus der Erntemaschine 23 herausgefördert. Der weitere Erntegutreststrom überquert dabei einen weiteren Verlustkornzähler 41, welcher ebenfalls als die Sensoranordnung 21 ausgeführt ist. Durch die Reinigungsvorrichtung 40 separiertes, gereinigtes Korn wird mittels eines Kornelevators in einen Korntank gefördert.

Der dargestellte Mähdrescher 1 weist zudem eine Überkehrschnecke 42 auf, welche einen von der Reinigungsvorrichtung 40 abgeschiedenen, nicht vollständig ausgedroschenen Überkehrerntegutstrom erneut der Dreschvorrichtung 27 zuführt. Dieser Überkehrerntegutstrom überquert bei seinem Weg zur Überkehrschnecke 42 einen Überkehr-Kornzähler 43, welcher ebenfalls als Sensoranordnung 21 ausgeführt ist. Von der Überkehrschnecke 42 wird der Überkehrerntegutstrom einem Überkehrelevator zugeführt, welcher die Überkehr der Dreschvorrichtung 27 zuführt.

In Fig. 3 ist schematisch und exemplarisch ein Prinzipaufbau einer aus dem Stand der Technik bekannten Sensoranordnung 50 dargestellt. Die als kapazitive Sensoranordnung ausgeführte Sensoranordnung 50 weist ein Gehäuse 51 auf, in welchem Komponenten der Sensoranordnung 50 schichtweise und räumlich beabstandet zueinander angeordnet sind. Das Gehäuse 51 weist ein Kontaktflächenelement 52 auf, über welche Erntegut hinwegströmt. Das Kontaktflächenelement 52 besteht aus einem abrasionsbeständigen Material. Im Inneren des Gehäuses 51 sind unterhalb des Kontaktflächenelements 52 und mit Abstand zu dieser Leiterplatten 53 mit Elektroden angeordnet. Die Elektroden auf den Leiterplatten 53 sind durch Leitungen 54 mit einer unterhalb der Leiterplatten 53 angeordneten, auf einer weiteren Leiterplatte angeordneten Auswerteschaltung 55 verbunden. Die Leiterplatte mit der Auswerteschaltung 55 ist durch weitere Leitungen 54 mit einer darunterliegenden plattenförmigen Steckeraufnahme 56 verbunden. An der Steckeraufnahme 56 ist ein Stecker 58 angeordnet, der aus dem Gehäuse 51 herausragt. Die Unterseite des Gehäuses 51 ist durch eine Abdeckung 57 verschlossen. Die aus einem Mehrleiterplatten-Aufbau bestehende Sensoranordnung 50 sieht eine räumliche Trennung der Elektroden 53, der Auswerteschaltung 55 und des dem Verschleißschutz dienenden Kontaktflächenelements 52 vor. Aus diesem Mehrleiterplatten-Aufbau resultieren mehrere Nachteile. So ist Bauraum Sensoranordnung 50 erhöht.

Es bedarf des Bondings zwischen den verschiedenen Leiterplatten. Es ist ein weitreichender Verguss der Komponenten der Sensoranordnung 50 notwendig. Insbesondere ist der Abstand zwischen dem Erntegut, welches das Kontaktflächenelement 52 überströmt und den Elektroden auf den Leiterplatte 53 groß und unterliegt starken Montagetoleranzen. Dies führt zu einem erhöhten Kalibrieraufwand. Ein weiterer Aspekt ist, dass die Feuchtigkeitsabsorption in den Leiterplatten 53 der Elektroden zu Messunsicherheiten führen kann. Weiterhin können Spannungen zwischen den Elektroden und dem Kontaktflächenelement 52 zu Fehlern führen

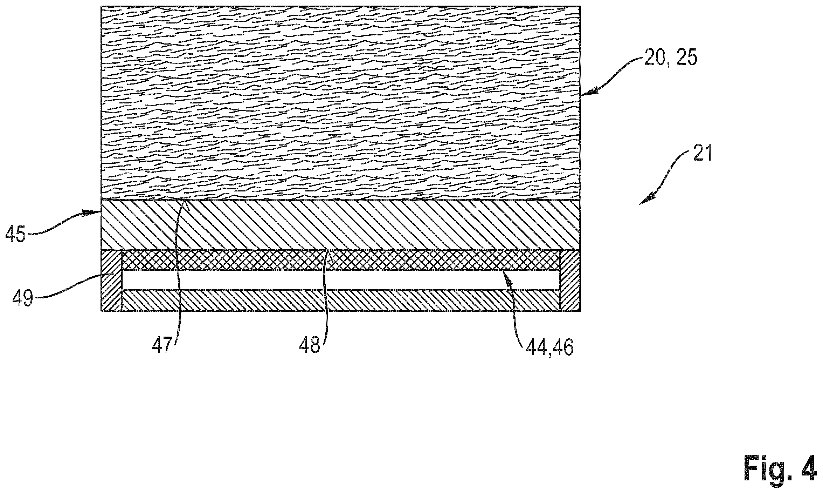

In Fig. 4 ist schematisch und exemplarisch ein Prinzipaufbau der erfindungsgemäßen Sensoranordnung 21 dargestellt. Die erfindungsgemäße Sensoranordnung 21 umfasst zumindest ein Sensorelement 44, welches auf einem plattenförmigen Träger 45 angeordnet ist, sowie eine Auswerteschaltung 46 zur Auswertung von Signalen, welche das zumindest eine Sensorelement 44 aufgrund eines physischen Kontakts des Trägers 45 mit dem Erntegutstrom 20, 25 generiert. Dazu ist der Träger 45 als eine keramikhaltige Platte mit einer Kontaktfläche 47, welche im Kontakt mit dem Erntegut steht, und einer der Kontaktfläche 47 abgewandten Sensorfläche 48 ausgeführt, wobei auf der dem Erntegut abgewandten Sensorfläche 48 des Trägers 45 das zumindest eine Sensorelement 44 und die Auswerteschaltung 46 an dieser angeordnet sind. Als Material für den Träger 45 kommt insbesondere eine technische Keramik zur Verwendung.

Das zumindest eine Sensorelement 44 ist als zwei zueinander beabstandete Elektroden ausgeführt. Das zumindest eine Sensorelement 44 und die Auswerteschaltung 46 sind als eine Leiterplatine ausgeführt. Erfindungsgemäss sind das zumindest eine Sensorelement 44 und die Auswerteschaltung 46 in die Sensorfläche 48 des 45 Trägers integriert. Hierzu ist auf der Sensorfläche 48 des 45 Trägers eine Kupferschicht aufgebracht, in welcher das zumindest eine Sensorelement 44 und die Auswerteschaltung 46 durch einen Bearbeitungsprozess ausgebildet werden.

Das Verfahren zum Herstellen der Sensoranordnung 21 umfasst die folgenden Verfahrensschritte:
- Herstellen des aus einem keramischen Material bestehenden plattenförmigen Träger 45 mit einer Kontaktfläche 47 und einer der Kontaktfläche 47 gegenüberliegenden Sensorfläche 48,
- Aufbringen einer Kupferschicht auf der Sensorfläche 48 des plattenförmigen Trägers 45 durch einen chemischen Bearbeitungsprozess, und
- Bearbeiten der Kupferschicht zur Ausbildung des zumindest einen Sensorelements 44 und einer Auswerteschaltung 46.

Das Bearbeiten der Kupferschicht, in welcher das zumindest eine Sensorelement 44 und die Auswerteschaltung 46 ausgebildet werden, erfolgt bevorzugt durch Ätzen oder Laserablation.

Wesentlich ist, dass durch den reduzierten Abstand zwischen dem überströmenden Erntegut und den Elektroden des zumindest einen Sensorelements 44 die Sensitivität der Messeinrichtung zunimmt und die Auflösung bei einer lokalen Erfassung der Permittivität erhöht werden kann. Die hierfür notwendigen Elektroden können verkleinert oder aber die Anforderungen an die Auswerteelektronik kann gesenkt werden.

Der Träger 45 kann in ein Gehäuse 49 eingeklebt werden. Mit Hinterguss des Trägers 45 auf der der Kontaktfläche 47 abgewandten Sensorfläche 48 ist auch ein direkter Einbau in Komponenten der Arbeitsorgane der Erntemaschine 1 denkbar, welche aufgrund der Bauhöhe von aus dem Stand der Technik bekannten Sensoranordnungen nicht zugänglich sind. Durch die in der Regel guten, materialabhängigen Wärmeeigenschaften des Trägers 45 unterliegt der Einbauort in der Erntemaschine 1 nur geringen Restriktionen.

Beabstandet zum plattenförmigen Träger 45 ist ein elektrischer Anschluss, in Form eines Steckers oder dergleichen, in dem den Träger 45 aufnehmenden Sensorgehäuse 49 angeordnet, um eine Leitung an die Sensoranordnung 21 anzuschließen.

### Bezugszeichenliste

| | | | |
|---|---|---|---|
| 1 | Erntemaschine | 34 | Rücklaufboden |
| 2 | Vorsatzgerät | 35 | Vorbereitungsboden |
| 3 | Einzugsorgan | 36 | Verlustkornzähler |
| 4 | Einzugswalze | 37 | Siebebene |
| 5 | Einzugswalze | 38 | Siebebene |
| 6 | Häckselvorrichtung | 39 | Gebläse |
| 7 | Häckseltrommel | 40 | Reinigungsvorrichtung |
| 8 | Häckselmesser | 41 | Verlustkornzähler |
| 9 | Gegenschneide | 42 | Überkehrschnecke |
| 10 | Förderkanal | 43 | Verlustkornzähler |
| 11 | Nachbearbeitungsvorrichtung | 44 | Sensorelement |
| 12 | Nachbeschleunigungsorgan | 45 | Träger |
| 13 | Überladeeinrichtung | 46 | Auswerteschaltung |
| 14 | Auswurfklappe | 47 | Kontaktfläche |
| 15 | Schichthöhensensor | 48 | Sensorfläche |
| 16 | Fahrerkabine | 49 | Gehäuse |
| 17 | Steuerungsvorrichtung | 50 | Sensoranordnung |
| 18 | Speichereinheit | 51 | Gehäuse |
| 19 | Recheneinheit | 52 | Kontaktflächenelement |
| 20 | Erntegutstrom | 53 | Leiterplatte |
| 21 | Sensoranordnung | 54 | Leitung |
| 22 | Feldhäcksler | 55 | Auswerteschaltung |
| 23 | Mähdrescher | 56 | Steckeraufnahme |
| 24 | Vorsatzgerät | 57 | Abdeckung |
| 25 | Erntegutstrom | 58 | Stecker |
| 26 | Schrägförderer | | |
| 27 | Dreschvorrichtung | | |
| 28 | Dreschkorb | | |
| 29 | Beschleunigungstrommel | | |
| 30 | Dreschtrommel | | |
| 31 | Umlenktrommel | | |
| 32 | Hordenschüttler | | |
| 33 | Abscheidevorrichtung | | |

## Patentansprüche

1. Sensoranordnung (21) zur Erfassung wenigstens einer Guteigenschaft eines von einer selbstfahrenden Erntemaschine aufgenommenen und diese durchlaufenden Erntegutes, umfassend zumindest ein Sensorelement (44), welches auf einem plattenförmigen Träger (45) angeordnet ist, sowie eine Auswerteschaltung (46) zur Auswertung von Signalen, welche das zumindest eine Sensorelement (44) aufgrund eines physischen Kontakts des Trägers (45) mit dem Erntegut generiert, wobei der Träger (45) als eine keramikhaltige Platte mit einer Kontaktfläche (47), welche im Kontakt mit dem Erntegut steht, und einer der Kontaktfläche (47) abgewandten Sensorfläche (48) ausgeführt ist, **dadurch gekennzeichnet, dass** auf der dem Erntegut abgewandten Sensorfläche (48) des Trägers (45) das zumindest eine Sensorelement (44) und die Auswerteschaltung (46) an dieser angeordnet sind, wobei das zumindest eine Sensorelement (44) und die Auswerteschaltung (46) in die Sensorfläche (48) des Trägers (45) integriert sind.

2. Sensoranordnung (21) nach Anspruch 1, **dadurch gekennzeichnet, dass** das zumindest eine Sensorelement (44) als zwei zueinander beabstandete Elektroden ausgeführt ist.

3. Sensoranordnung (21) nach Anspruch 1 oder 2, **dadurch gekennzeichnet, dass** das zumindest eine Sensorelement (44) und die Auswerteschaltung (46) als eine Leiterplatine ausgeführt sind.

4. Sensoranordnung (21) nach einem der Ansprüche 1 bis 3, **dadurch gekennzeichnet, dass** beabstandet zum plattenförmigen Träger (45) ein elektrischer Anschluss in einem den Träger aufnehmenden Sensorgehäuse (49) angeordnet ist, um eine Leitung an die Sensoranordnung (21) anzuschließen.

5. Selbstfahrende Erntemaschine (1), die zur Aufnahme und Bearbeitung von Erntegut mittels verschiedener an oder in der Erntemaschine (1) angeordneter Arbeitsorgane eingerichtet ist, wobei in der Erntemaschine (1) zumindest eine mit dem Erntegut in physischen Kontakt kommende Sensoranordnung (21) zur Erfassung eines Erntegutparameters angeordnet ist, **dadurch gekennzeichnet, dass** die zumindest eine Sensoranordnung (21) nach einem der vorangehenden Ansprüche 1 bis 5 ausgebildet ist.

6. Selbstfahrende Erntemaschine (1) nach Anspruch 5, **dadurch gekennzeichnet, dass** die zumindest eine Sensoranordnung (21) als kapazitive Sensoranordnung ausgeführt ist.

7. Selbstfahrende Erntemaschine (1) nach Anspruch 5 oder 6, **dadurch gekennzeichnet, dass** die Erntemaschine als Feldhäcksler (22) oder als Mähdrescher (23) ausgeführt ist.

8. Selbstfahrende Erntemaschine (1) nach Anspruch 7, **dadurch gekennzeichnet, dass** die zumindest eine Sensoranordnung (21) in wenigstens einer Wandung eines Einzugskanals (2), eines Förderschachts (10) und/oder eines Auswurfkrümmers (13) des Feldhäckslers (22) angeordnet ist.

9. Selbstfahrende Erntemaschine (1) nach Anspruch 7, **dadurch gekennzeichnet, dass** die zumindest eine Sensoranordnung (21) in wenigstens einer Wandung eines Schrägförderers (26), eines Kornelevators und/oder eines Überkehrelevators des Mähdreschers (23) angeordnet ist und/oder dass die zumindest eine Sensoranordnung (21) einer Dreschvorrichtung (27), einer Abscheidevorrichtung (33) und/oder einer Reinigungsvorrichtung (40) des Mähdreschers (23) zugeordnet ist.

10. Selbstfahrende Erntemaschine (1) nach einem der Ansprüche 5 bis 9, **dadurch gekennzeichnet, dass** die zumindest eine Sensoranordnung (21) zur Detektion einer Erntegutfeuchtigkeit, eines Erntegutdurchsatzes, einer Erntegutdichte und/oder einer Querverteilung eingerichtet ist.

11. Selbstfahrende Erntemaschine (1) nach einem der Ansprüche 6 bis 11, **dadurch gekennzeichnet, dass** der Träger der Sensoranordnung (21) in ein Sensorgehäuse (49) oder unmittelbar in eine mit dem Erntegut in Berührung kommende Fläche eines der Arbeitsorgane integriert ist.

## Claims

1. Sensor arrangement (21) for detecting at least one material property of a crop received by a self-propelled harvester and passing through the harvester, comprising at least one sensor element (44), which is arranged on a plate-like carrier (45), and an evaluation circuit (46) for evaluating signals, which generates the at least one sensor element (44) on account of physical contact between the carrier (45) and the crop, wherein the carrier (45) is embodied as a ceramic-containing plate with a contact surface (47), which is in contact with the crop, and a sensor surface (48) facing away from the contact surface (47), **characterized in that** the at least one sensor element (44) and the evaluation circuit (46) are arranged on the sensor surface (48) of the carrier (45) facing away from the crop, wherein the at least one sensor element (44) and the evaluation circuit (46) are integrated into the sensor surface (48) of the carrier (45) .

2. Sensor arrangement (21) according to Claim 1, **characterized in that** the at least one sensor element (44) is embodied as two electrodes which are spaced apart from each other.

3. Sensor arrangement (21) according to Claim 1 or 2, **characterized in that** the at least one sensor element (44) and the evaluation circuit (46) are embodied as a printed circuit board.

4. Sensor arrangement (21) according to any of Claims 1 to 3, **characterized in that** an electrical connection is arranged in a sensor housing (49) receiving the carrier in a manner spaced apart from the plate-like carrier (45) in order to connect a line to the sensor arrangement (21).

5. Self-propelled harvester (1) which is configured for receiving and processing crop by means of various working members arranged on or in the harvester (1), wherein at least one sensor arrangement (21) that comes into physical contact with the crop is arranged in the harvester (1) for detecting a crop parameter, **characterized in that** the at least one sensor arrangement (21) is designed according to any of the preceding Claims 1 to 4.

6. Self-propelled harvester (1) according to Claim 5, **characterized in that** the at least one sensor arrangement (21) is embodied as a capacitive sensor arrangement.

7. Self-propelled harvester (1) according to Claim 5 or 6, **characterized in that** the harvester is embodied as a forage harvester (22) or as a combine harvester (23).

8. Self-propelled harvester (1) according to Claim 7, **characterized in that** the at least one sensor arrangement (21) is arranged in at least one wall of an intake duct (2), a conveying shaft (10) and/or a discharge chute (13) of the forage harvester (22).

9. Self-propelled harvester (1) according to Claim 7, **characterized in that** the at least one sensor arrangement (21) is arranged in at least one wall of an inclined conveyor (26), a grain elevator and/or a returns elevator of the combine harvester (23) and/or **in that** the at least one sensor arrangement (21) is assigned to a threshing device (27), a separating device (33) and/or a cleaning device (40) of the combine harvester (23).

10. Self-propelled harvester (1) according to any of Claims 5 to 9, **characterized in that** the at least one sensor arrangement (21) is configured for detecting a crop moisture content, a crop throughput, a crop density and/or a lateral distribution.

11. Self-propelled harvester (1) according to any of Claims 6 to 10, **characterized in that** the carrier of the sensor arrangement (21) is integrated into a sensor housing (49) or directly into a surface of one of the working members that comes into contact with the crop.

## Revendications

1. Agencement de capteur (21) pour la détection d'au moins une propriété de produit de récolte d'un produit de récolte ramassé par une machine de récolte automotrice et traversant celle-ci, comprenant au moins un élément capteur (44) qui est disposé sur un support (45) en forme de plaque, ainsi qu'un circuit d'évaluation (46) pour l'évaluation de signaux que ledit au moins un élément capteur (44) génère en raison d'un contact physique du support (45) avec le produit de récolte, le support (45) étant réalisé sous la forme d'une plaque contenant de la céramique avec une surface de contact (47) qui est en contact avec le produit de récolte, et une surface de capteur (48) opposée à la surface de contact (47), **caractérisé en ce que**, sur la surface de capteur (48) du support (45) opposée au produit de récolte, ledit au moins un élément capteur (44) et le circuit d'évaluation (46) sont disposés sur celle-ci, ledit au moins un élément capteur (44) et le circuit d'évaluation (46) étant intégrés dans la surface de capteur (48) du support (45).

2. Agencement de capteur (21) selon la revendication 1, **caractérisé en ce que** ledit au moins un élément capteur (44) est réalisé sous la forme de deux électrodes espacées l'une de l'autre.

3. Agencement de capteur (21) selon la revendication 1 ou 2, **caractérisé en ce que** ledit au moins un élément capteur (44) et le circuit d'évaluation (46) sont réalisés sous la forme d'un circuit imprimé.

4. Agencement de capteur (21) selon l'une des revendications 1 à 3, **caractérisé en ce qu'**un raccordement électrique est disposé à distance du support en forme de plaque (45) dans un boîtier de capteur (49) recevant le support, afin de raccorder un conduit à l'agencement de capteur (21).

5. Machine de récolte automotrice (1) conçue pour ramasser et traiter un produit de récolte au moyen de différents organes de travail disposés sur ou dans la machine de récolte (1), au moins un agencement de capteur (21) venant en contact physique avec le produit de récolte étant disposé dans la machine de récolte (1) pour la détection d'un paramètre de produit de récolte, **caractérisée en ce que** ledit au moins un agencement de capteur (21) est réalisé selon l'une des revendications précédentes 1 à 4.

6. Machine de récolte automotrice (1) selon la revendication 5, **caractérisée en ce que** ledit au moins un agencement de capteur (21) est réalisé sous la forme d'un agencement de capteur capacitif.

7. Machine de récolte automotrice (1) selon la revendication 5 ou 6, **caractérisée en ce que** la machine de récolte est réalisée sous la forme d'une ensileuse (22) ou d'une moissonneuse-batteuse (23).

8. Machine de récolte automotrice (1) selon la revendication 7, **caractérisée en ce que** ledit au moins un agencement de capteur (21) est disposé dans au moins une paroi d'un canal d'alimentation (2), d'un puits de transport (10) et/ou d'une goulotte d'éjection (13) de l'ensileuse (22).

9. Machine de récolte automotrice (1) selon la revendication 7, **caractérisée en ce que** ledit au moins un agencement de capteur (21) est disposé dans au moins une paroi d'un convoyeur incliné (26), d'un élévateur à grains et/ou d'un élévateur à otons de la moissonneuse-batteuse (23) et/ou **en ce que** ledit au moins un agencement de capteur (21) est associé à un dispositif de battage (27), un dispositif de séparation (33) et/ou un dispositif de nettoyage (40) de la moissonneuse-batteuse (23).

10. Machine de récolte automotrice (1) selon l'une des revendications 5 à 9, **caractérisée en ce que** ledit au moins un agencement de capteur (21) est conçu pour la détection d'une humidité de produit de récolte, d'un débit de produit de récolte, d'une densité de produit de récolte et/ou d'une répartition transversale.

11. Machine de récolte automotrice (1) selon l'une des revendications 6 à 10, **caractérisée en ce que** le support de l'agencement de capteur (21) est intégré dans un boîtier de capteur (49) ou directement dans une surface de l'un des organes de travail venant en contact avec le produit de récolte.
